# EUROPEAN PATENT SPECIFICATION

(11) **EP 2 760 953 B1**
(45) Date of publication and mention of the grant of the patent: **21.12.2016**
(21) Application number: 12769570.8
(22) Date of filing: 20.09.2012
(51) Int. Cl.: C09J 5/08, C08J 9/12, A61F 13/02, B29C 44/08, B29C 44/26

(54) **METHODS FOR MAKING A FOAMED ADHESIVE CONTAINING CLOSED CELLS**
VERFAHREN ZUR HERSTELLUNG EINES GESCHÄUMTEN HAFTMITTELS MIT GESCHLOSSENEN ZELLEN
PROCÉDÉS DE FABRICATION D'UN ADHÉSIF MOUSSE CONTENANT DES CELLULES FERMÉES

(30) Priority: 28.09.2011 US 201113246921; 22.12.2011 US 201113334638
(43) Date of publication of application: 06.08.2014
(73) Proprietor: Johnson & Johnson Consumer Inc., Skillman, NJ 08558 (US)
(72) Inventor: LUNDE, Erik, Morganville, New Jersey 07751 (US); PATEL, Bharat D., Princeton, New Jersey 08540 (US); PATEL, Ravi, Florence, New Jersey 08518 (US)
(74) Representative: Carpmaels & Ransford LLP
(86) International application number: PCT/US2012/056288
(87) International publication number: WO 2013/048862

(56) References cited:
- DE-A1- 10 305 224
- DE-A1- 19 620 107
- DE-A1-102009 015 233
- US-A- 5 342 858

## Description

### FIELD OF THE INVENTION

This invention relates to methods of making foamed adhesives for use in a dressing for topical application to the body and that includes a carrier substrate and foamed adhesive layer applied to the carrier substrate, where the foamed adhesive layer includes a plurality of closed cells that include an inert gas disposed therein and which are substantially homogeneously distributed throughout the foamed adhesive layer.

### BACKGROUND OF THE INVENTION

Adhesives for dressings that may be placed in contact with skin, either directly or indirectly, are known in the art. Such dressings include adhesive bandages for direct application to wounds in the skin, as well as absorbent articles, such as sanitary napkins, that are indirectly placed in contact with the skin via attachment to undergarments or articles of clothing. Such adhesives include hot melt adhesives.

Hot melt adhesives are applied to carrier substrates in molten form and typically will form a solid layer on the substrate upon cooling. Such adhesive compositions and layers typically will be substantially void of bubbles or pores that may contain air or other gases. Other hot melt adhesives, such as those described in U.S. Pat. No. 6,383,630, may include open cells or pores, and form open-celled adhesive layers which are said to exhibit good permeability to air and water vapor. As described therein, the adhesive composition is "applied not to the entire area of at least one side" of the carrier substrate. The adhesive layer preferably is patterned in a dome form. The adhesive layers in dressings exemplified therein are applied to the carrier substrate by thermal screen printing.

Adhesive dressings that include a layer of a foamed adhesive composition prepared by the methods of the present invention permit reduction of the amount of adhesive composition that must be applied to the carrier substrate, thus resulting in cost savings versus dressings that use a substantially similar non-foamed adhesive composition, while maintaining adhesion and bulk properties necessary for use in such dressings.

US5342858 (A) describes an elastomeric, hot-melt adhesive foam. The adhesive foam preferably comprises an elastomeric, hot-melt adhesive material formed from: (a) about 15% to about 60%, by weight of the adhesive material, of an A-B-A block copolymer, in which the A block is derived from styrene and the B block is derived from butadiene or isoprene; (b) about 30% to about 70%, by weight of the adhesive material, of an aromatic modified hydrocarbon resin which associates with both the B block and A blocks of the A-B-A block copolymer; and (c) 0 to about 30%, by weight of the adhesive material, of a processing oil. The proportions of the components (a), (b), and (c) are selected such that the elastomeric, hot-melt adhesive material has a viscosity of less than about 200,000 centipoise at 325 DEG F (163°C) and an elastomeric retention value of at least 75%. The adhesive material is preferably pressure-sensitive so as to allow lamination of the solified foam comprising the adhesive material with components of absorbent articles without the need for external bonding agents. Also disclosed is a method of making the elastomeric adhesive foam and of elasticizing structures and absorbent articles with the foam.

### SUMMARY OF THE INVENTION

The invention relates to a method for making a foamed adhesive composition for use in dressings suitable for topical application to the body, according to claim 1. The invention further relates to foamed adhesive compositions prepared by such methods.

### BRIEF DESCRIPTION OF THE FIGURES

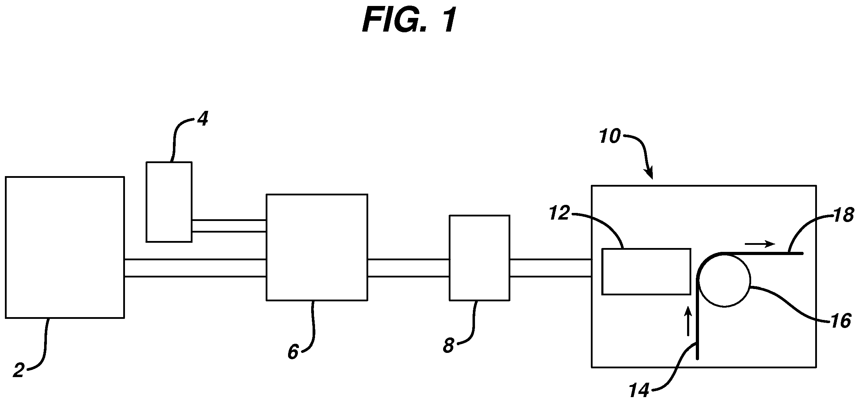

FIG. 1 is a schematic of a process and equipment according to the present invention

### DETAILED DESCRIPTION

The invention relates to methods for making foamed adhesive compositions suitable for use in dressings that are topically applied to or otherwise placed in contact with the body for use in various applications. Such dressings include, without limitation, adhesive bandages for use in wound care, e.g. for application to cuts and scrapes in the skin, and feminine sanitary protection products, e.g. napkins and liners. Such dressings will include a carrier substrate having at least one side to which a foamed adhesive composition as described herein is applied to form an adhesive layer for the purpose of securing the dressing in place relative to the body. The adhesive layer will contain a proportion of inert gas contained in a plurality of closed cells, which cells are substantially homogenously distributed throughout the adhesive layer.

Foamed adhesive compositions which can be employed in such dressings may be hot-melt adhesives having a plurality of closed cells that contain an inert gas disposed therein and which are substantially homogenously dispersed throughout the adhesive. By "substantially homogenously dispersed", it is meant that the number and volume of closed cells dispersed in any portion, e.g. volume, of the foamed adhesive composition or foamed adhesive layer is approximately the same. By "closed cell", it is meant that the cells are predominantly isolated from one another and are bounded by a definite/intact cell boundary of continuous adhesive material, i.e. a substantially continuous cell wall, such that the inert gas is encapsulated and retained within the cells.

The closed cells in the foamed adhesive composition may have an ovoid, spherical or circular structure. The closed cell in the foamed adhesive typically will have a maximum cell dimension, e.g. maximum average cell diameter, of about 10 micron, or about 5 micron. The cell dimension may range from about 1 to about 10 micron or from about 2 to about 5 micron, depending on the particular application. While it is possible for the foamed adhesive compositions to include some relatively minor level of open cells, the cell volume in the adhesive composition is predominately made up of closed cells. Typically, the total number of cells in the adhesive composition and/or the adhesive layer will contain less than 10% of open cells, and will comprise about 90% or more of closed cells, and even more typically will comprise about 95% or more of closed cells.

The cell boundary prevents the free flow of inert gas from the closed cell in the foamed adhesive, allowing only minimal permeation. Furthermore, due to the closed cell structure, the inert gas will not easily escape from the closed cells upon application of pressure associated with applying the dressing to the carrier substrate. In addition, the closed-cell nature of the foamed adhesive provides for a highly effective air barrier, low moisture vapor permeability and excellent resistance to water.

The foamed adhesive compositions prepared according to the present invention may be based on materials that are used to prepare adhesives used in conventional dressings, including without limitation, natural and/or synthetic rubbers and/or on other synthetic polymers, such as acrylates, methacrylates, polyurethanes, polyolefins, polyvinyl derivatives, polyesters or silicones, or blends thereof. The adhesive compositions may also include appropriate additives, such as adhesion resins, plasticizers, stabilizers and other auxiliary components where necessary or desired.

Gases used to prepare adhesive compositions utilized in the present invention may be inert gases selected from the group consisting of nitrogen, carbon dioxide, noble gases, hydrocarbons; and air. In the case of air, anti-oxidants may be added to the adhesives. Because of the inert nature of the gases, there are no reactions between the adhesive compositions and the gases in the foamed adhesives.

For a foamed adhesive to maintain bulk adhesive properties that are effective for use of dressings of the present invention in the intended applications, and to maintain sufficient adhesive properties, the foamed adhesive needs to be prepared with the appropriate degree of foaming. The degree of foaming, i.e. the proportion of gas, e.g. volume, dispersed in and distributed throughout the adhesive composition is effective to provide the adhesive layer the requisite adhesive properties. The degree of foaming in foamed adhesive compositions used in dressings according to the present invention may be from about 15% to 70%, by volume, or from about 20% to about 60% by volume, or about 20% to 40% by volume. The density of foamed adhesive compositions used in dressings of the present invention may range from about 0.14 to 0.80 g/cm³, or from about 0.19 to about 0.55 g/cm³, or from about 0.19 to about 0.38 g/cm³.

The foamed adhesives are prepared at the temperature just above the melting point of the adhesive material. The temperature may range from about 135°C (275°F) to about 190°C (375°F) or from about 150°C (300°F) to about 157°C (315°F). Typically, the processing temperature of the foamed adhesives to be used in dressings for use in wound care ranges from about 150°C (300°F) to about 177°C (350°F), and the temperature is slightly lower for foamed adhesives to be used in dressings for use in sanitary protection. The mixing is conducted at a relatively high speed. In addition to temperature and speed of mixing, other conditions of preparing the closed-cell foamed adhesive compositions must be effective to provide the closed-cell structures substantially homogeneously dispersed therein.

In order to prepare foamed adhesives with predominately closed cells, as described above, a particularly suitable process for producing the foamed adhesives is employed as described herein. In this process, the pressure sensitive adhesive is first melted at a temperature greater than the melting point of the adhesive to form a molten adhesive composition. The molten adhesive is then mixed in a first mixer comprising means for mixing the molten adhesive and gas, e.g. a rotating mixing blade, with dry inert gases, such as nitrogen, air or carbon dioxide, in different proportions by volume, e.g. 10-80%, under high pressures to form a first foamed adhesive composition. The gas, e.g. N₂, is pumped into the molten adhesive at high pressure, e.g. from about 3.45 MPa (500 psi) to about 10.3 MPa (1500 psi), such that the adhesive pressure is approximately 2.07-4.14 MPa (300-600 psi) in the first mixer, where the coarse mixing occurs. Such first foamed adhesive compositions and methods of preparing such are conventional and additional mixing conditions for forming the first foamed adhesive compositions will be readily ascertainable by those skilled in the art once having the benefit of this disclosure.

The first foamed adhesive composition is subsequently pumped into the second mixer, also comprising means for mixing the first foamed adhesive composition, e.g. a rotating blade or other mixing structure, in order to continue fine mixing to yield a second foamed adhesive composition comprising a substantially homogeneous distribution of closed cells. Those skilled in the art will recognize that means for mixing the foamed adhesive compositions may include structures other than rotating mixing blades. Additional gas optionally may be added to the second mixer. Additional mixers may be used as required. The external pressure, i.e. the die pressure, is maintained at from 1.21 MPa (175 psi) to 2.24 MPa (3.25 psi).

Mixing conditions in the secondary mixer must be effective to produce the adhesive composition comprising a homogenous distribution of closed cells having the inert gas contained therein. Such conditions include, without limitation, the pump speed, i.e. the speed of the rotating mixing blade, and the pressure differential (dP) between the internal pressure of the first adhesive composition entering the second mixer and the external pressure of the second foamed adhesive composition exiting the second mixer. The internal pressure of the first foamed adhesive composition entering the second mixer is greater than the external pressure of the second foamed adhesive composition exiting the second mixer, thereby providing the pressure differential (dP). Typically, the internal pressure of the first foamed adhesive composition entering the second mixer may be from about 2.76 MPa (400 psi) to about 2.93 MPa (425 psi). The external pressure of the second foamed composition exiting the second mixer is from 1.21 MPa (175 psi) to 2.24 MPa (325 psi), or from 1.24 MPa (180 psi) to 2.07 MPa (300 psi). The minimum dP should be effective to provide the closed cells in the second adhesive composition. However, excessive dP may lead to instability of the foam in the adhesive composition. The dP of the internal vs. external pressure of the second mixer typically will be about 0.79 MPa (115 psi) or greater.

The pump, i.e. mixing blade, speed varies from equipment to equipment, but it must be sufficiently high to yield a substantially homogeneous distribution of the closed cells containing the gas in the adhesives. One such mixer is a Nordson^{®} GP 200 which includes a high shearing blade. When utilizing the Nordson^{®} GP 200, the speed of rotation of the mixing blade may range from about 20 to about 30 rpm. One skilled in the art will recognize that other mixers may utilize different mixing speeds. Further, to maintain the closed nature of the cell, the viscosity of the molten adhesives should be in the range of 1,000-50,000cps at the temperature where the mixing of the gases and molten adhesives occurs to sustain the close cells.

The second foamed adhesive composition is then transferred through insulated (i.e. temperature controlled) pipes to the dispensing station and dispensed through a slot die for example, onto the carrier substrate. The slot die can be designed to dispense various patterns to the surface of the substrate, whether over the entire or partial area of the substrate.

By optimizing mixing conditions in the second mixer and providing the second foamed adhesive composition comprising the homogenous distribution of closed cells, the density of the adhesive composition is decreased such that less adhesive by weight is used in the foamed compositions, while maintaining adhesive properties necessary for effective performance in the intended use. The calculated density reduction for the second foamed adhesive compositions, i.e. the weight percent reduction of adhesive in a constant volume of the second foamed adhesive composition, may range from about 10 percent to about 50 percent, or from about 20 percent to about 40 percent.

As shown in FIG. 1, one process of the invention utilizes adhesive storage **2** that feeds molten adhesive to primary mixer **6.** Inert gas, e.g. nitrogen, storage **4** provides nitrogen to primary mixer **6** connected therewith where a first foamed adhesive is formed. The first foamed adhesive is then transferred from primary mixer **6** to secondary mixer **8** where further mixing produces a second foamed adhesive composition having a substantially homogenous distribution of closed cells containing the nitrogen enclosed therein. The second foamed adhesive composition is then transferred from secondary mixer **8** to application system **10.** Application system **10** comprises adhesive applicator **12** that applies foamed adhesives mixed in secondary mixer **8** to the uncoated fabric substrate **14** supported by coating roll **16,** yielding coated fabric substrate **18.**

The foamed adhesive made according to the invention may be applied to the uncoated fabric substrate **14** by brushing. In an alternative embodiment, the foamed adhesive may be applied to the uncoated fabric substrate **14** by spraying. Yet in another embodiment, the foamed adhesive made according to the invention may be applied to the uncoated fabric substrate **14** by dripping. One skilled in the art will be able to readily ascertain other application mechanism that may be employed once having the benefit of this disclosure.

Carrier substrates used in the dressings may be any substrate conventionally used in applications in which the dressings may be used. The foamed adhesive is applied to the substrate in order to secure the dressing in place in relationship to the surface of the body to which the dressing is applied. The carrier substrates will have a first side and a second side, opposite the first side, to which the foamed adhesive composition is applied to form the foamed adhesive layer. The adhesive layer may be continuous, i.e. covering substantially the entire area of the second side of the substrate to which it is applied. The adhesive layer also may be discontinuous, in which case the foamed adhesive may be applied in a pattern, or around the periphery of the second side of the substrate, and the like, thus having adhesive areas and non-adhesive areas. The total adhesive area must be sufficient to secure the dressing in place.

In the case of adhesive bandages, the second side of the substrate having the foamed adhesive layer thereon will be placed directly on the skin, securing the bandage directly to the skin. An absorbent pad may be applied onto the surface of the adhesive layer facing the skin, in which case the adhesive layer may serve to adhere the bandage to the skin, as well as to adhere the absorbent pad to the carrier substrate, e.g. a backing layer conventionally used in adhesive bandage applications.

In the case of a sanitary napkin embodiment, the carrier substrate may be used in conjunction with other absorbent pads or layers in order to provide requisite absorption of body fluids. The second side of the carrier substrate having the foamed adhesive layer applied thereto is then place in contact with the undergarment or other article of clothing to secure the napkin in place.

The substrates may be made from woven or knitted fabrics, elastic or inelastic materials, plastic films, paper, nonwovens fabrics, foam materials, or laminates thereof. Polymeric materials that may be used in preparation of the carrier substrate include, but are not limited to, polyethylene, polyolefinic films, coextruded polyolefinic films, polyurethane film, and PU/PVC foam backing. In certain adhesive bandage embodiments, the carrier substrate may be a smooth surface or aperture film, such as mono- or co-extruded polyolefin films, e.g. polyethylene or polypropylene, polyurethane or other thin films. Other substrates include a coarse, textured surface/topography as with woven or non-woven flexible fabrics made from, e.g. natural or synthetic fibers such as cotton, rayon, PET, nylon, polyurethane, etc. In embodiments where the dressing is a sanitary napkin, the carrier substrate may be a film, e.g. polyethylene, or a non-woven e.g. polypropylene.

The foamed adhesive compositions having the closed cells distributed there through are applied to the side of the carrier substrate that secures the dressing of the present invention in relationship to the body. As noted above, the side containing the foamed adhesive layer secures the adhesive bandage to the skin, while the side containing the foamed adhesive layer secures the sanitary napkins or liner to an article of clothing.

As in the foamed adhesive composition, the closed cells distributed throughout the foamed adhesive layer have a cell dimension that may range from about 1 to about 10 micron or from about 2 to about 5 micron. In some embodiments of the invention, the closed cell may be elongate in structure due to application of the foamed adhesive composition to the carrier substrate, in which case the cell dimension may be length and/or width of the cell. In other embodiments, the closed cell may be ovoid, spherical or circular in structure, in which case the cell dimension may be an average cell diameter. In certain embodiments the cell dimension has a maximum of about 10 micron, or in other embodiments, the cell dimension has a maximum of about 5 micron.

The thickness of the foamed adhesive layer may be from about 20 to about 200 micron or from about 30 to about 100 micron. The ratio of the thickness of the foamed adhesive layer to the average cell dimension may be from about 0.005 to about 0.50, or from about 0.03 to about 0.1. The foamed adhesive layer may have a basis weight ranging from about 20 to about 150 g/m² or from about 30 to about 100 g/m² or from about 30 to about 60 g/m². The density of the foamed adhesive layer may be from about 0.14 to about 0.80 g/cm³, or from about 0.19 to about 0.55 g/cm³, or from about 0.19 to about 0.38 g/cm³.

In preparation of the adhesive dressings made according to the present invention, the foamed adhesive composition as described herein are coated onto the side of the carrier substrate that will secure the dressing in place in relation to the body.

Adhesive dressings made according to the present invention exhibit a number of advantages. First, the amount of adhesive required to be used in the adhesive layer is considerably reduced by the presence of the closed cells filled with gases without adversely affecting the adhesion properties and bulk properties of the adhesive layer. This provides a significant savings to the manufacturer due to the reduced amount of adhesive composition actually used. Further, the foamed adhesive layer gives the adhesive dressing a soft and smooth feel, providing an improved comfort upon application.

The adhesive dressings typically have a static shear that is about 20% to about 80% of the static shear of an adhesive dressing comprising substantially the same, or the same, carrier substrate, and a layer of non-foamed adhesive composition that is substantially similar to the foamed adhesive composition. In certain embodiments the dressings of the invention will have a static shear of about 60%, or about 80% of the static shear of an adhesive dressing comprising substantially the same, or the same, carrier substrate, and a layer of non-foamed adhesive composition that is substantially similar to the foamed adhesive composition. The dressings may have a static shear of about 100 minutes or greater, or 300 minutes or greater, or about 500 minutes or greater, or about 1500 minutes or greater, each as determined by methods referenced and described herein below with a static load at 500g. As set forth in the claims, all static shear values are determined by the methods described in ASTM 6463, at a static load of 500g.

### Example 1:

A hot-melt adhesive composition with no foaming was applied to two different carrier substrates, one being a flexible, woven PET fabric (Control C1) and the other a polyolefin film having outer films of PE and a film of PE/acrylic blend disposed between the outer films (Control C2). Hot-melt adhesive compositions of varying degrees of foaming were applied to the same substrates as C1 and C2, respectively. The hot-melt adhesives were applied to the various carrier substrates at approximately 160°C. The resulting coated substrates, C1, C2, 1A, 1B, 1C and 1D, were then tested for 90° Adhesion and static shear utilizing standard test methods ASTM 3330 F and ASTM 6463, respectively. Results are presented in Table 1 below.

**Table 1**

| | **Flexible Fabric** | | | **PE Backing (Sheer)** | | |
|---|---|---|---|---|---|---|
| | **C1** | **1A** | **1B** | **C2** | **1C** | **1D** |
| Basis Weight (g/m²) | 58 | 40 | 28 | 50 | 38 | 25 |
| Foaming (Volume %) | 0 | 31 | 51 | | 24 | 50 |
| Thickness (mm) (mil) | 0.419 (16.5) | 0.302 (11.9) | 0.320 (12.6) | 0.152 (6.0) | 0.145 (5.7) | 0.135 (5.3) |
| 90⁰ Adhesion/Glass (N/m) (oz/in) | 0.134 (19) | 0.120 (17) | 0.099 (14) | 0.141 (20) | 0.071 (10) | 0.028 (4) |
| 90⁰ Adhesion/Backing (N/m) (oz/in) | 0.085 (12) | 0.049 (7) | 0.042 (6) | 0.240 (34) | 0.113 (16) | 0.056 (8) |
| Static Shear (min.) 0.75" x 0.75" x 500g (Static Load) | 545 | 308 | 195 | >6000 | >6000 | >6000 |
| Static Shear (min.) 0.75" x 0.75" x 750 g (Static Load) | 156 | 98 | n/a | Backing breaks - unable to measure | | |

As the results indicate, the woven fabric having a foamed adhesive layer containing about 30 and 50 % foaming (by volume) exhibited a static shear of 308 and 195 minutes at a static load of 500g, respectively. The polyolefin films with the same adhesive layer exhibited static shear that were immeasurable.

### Example 2:

A series of foamed adhesive compositions comprising a homogenous distribution of closed cells comprising nitrogen contained therein were prepared according to the present invention utilizing the Nordson^{®} GP 200 Precision Pump as described above. Process parameters for each run are shown in Table 2 below. As is shown in the Table, the calculated density reduction for the various process parameters ranged from about 10 percent to about 40 percent.

**Table 2**

| **Internal Pressure Second Mixer (MPa) (psi)** | **External Pressure Second Mixer (MPa) (psi)** | **Nitrogen Pressure First Mixer (MPa) (psi)** | **Mixing blade speed (rpm)** | **Applicator Slot width/shim thickness** | **Pan sample wt (g)** | **Calculated Density Reduction** | **dP** |
|---|---|---|---|---|---|---|---|
| 2.93 (425) | 1.99 (288) | 3.72 (540) | 26.1 | 4"/0.014" | 33 | **10%** | 137 |
| 2.86 (415) | 2.04 (296) | 3.72 (540) | 26.2 | 4"/0.014" | 33.3 | **10%** | 119 |
| 2.93 (425) | 2.01 (291) | 3.72 (540) | 26.2 | 4"/0.014" | 32.6 | **10%** | 134 |
| 2.86 (415) | 1.28 (185) | 3.72 (540) | 26.6 | 4"/0.014" | 29 | **20%** | 230 |
| 2.93 (425) | 1.34 (195) | 3.72 (540) | 26.1 | 4"/0.014" | 28.7 | **25%** | 230 |
| 2.76 (400) | 1.53 (222) | 3.72 (540) | 26.2 | 4"/0.014" | 24.3 | **33%** | 178 |
| 2.76 (400) | 1.25 (182) | 3.72 (540) | 20.4 | 4"/0.014" | 22.3 | **38%** | 218 |

## Claims

1. A method for making foamed adhesive compositions suitable for use in adhesive dressings for topical application to the body, the method comprising:
providing a molten adhesive composition,
combining an inert gas with said molten adhesive composition in a first mixer comprising means for mixing said inert gas and molten adhesive,
mixing said molten adhesive composition comprising said inert gas in the said mixer under conditions effective to form a first foamed adhesive composition; and
mixing said first foamed adhesive composition in a second mixer comprising a means for mixing said first foamed adhesive composition under conditions effective to provide a second foamed adhesive composition comprising a substantially homogenous distribution of closed cells comprising said inert gas contain therein,
wherein an internal pressure of said first foamed adhesive composition entering said second mixer is greater than an external pressure of said second foamed adhesive composition exiting said second mixer, thereby providing a pressure differential, wherein said internal pressure is from 2.59 MPa (375 psi) to 3.10 MPa (450 psi) and wherein said external pressure is from 1.21 MPa (175 psi) to 2.24 MPa (325 psi).

2. The method of claim 1 wherein said pressure differential is:
a) 0.79 MPa (115 psi) or greater.

3. The method of claim 1 wherein said internal pressure is from 2.76 MPa (400 psi) to 2.93 MPa (425 psi).

4. The method of claim 1 wherein said external pressure is from 1.24 MPa (180 psi) to 2.07 MPa (300 psi).

5. The method of claim 1 wherein the pressure of said inert gas entering said first mixer is greater than the pressure of said molten adhesive composition entering said first mixer.

6. The method of claim 5 wherein said pressure of said molten adhesive entering said first mixer is about the same as said internal pressure of said first foamed adhesive composition.

7. The method of claim 1 wherein said means for mixing said first adhesive composition in said second mixer is a rotating mixing blade.

8. The method of claim 7 wherein said rotating mixing blade rotates at from about 20 to about 30 rpm.

9. The method of claim 1 wherein said second foamed adhesive composition has a calculated density reduction of:
a) from 10 percent to 50 percent; or
b) from 20 percent to 40 percent.

10. The method of claim 1 wherein said closed cells are ovoid, spherical, circular or elongate in structure.

11. The method of claim 1 wherein said closed cells comprise a cell dimension selected from the group consisting of average diameter, length and width, preferably wherein said cell dimension is from 1 to 10 micron.

12. The method of claim 1 wherein said second foamed adhesive composition has a density from 0.14 to 0.80 g/cm³.

13. The method of claim 1 wherein said inert gas is selected from the group consisting of nitrogen, carbon dioxide, noble gases, hydrocarbons and air.

14. A foamed adhesive composition comprising a substantially homogenous distribution of closed cells comprising said inert gas contain therein prepared according to the method of claim 1, wherein the foam has a cell dimension selected from the group consisting of average diameter, length and width of from 1 to 10 micron.

## Patentansprüche

1. Verfahren zum Herstellen geschäumter Klebstoffzusammensetzungen, die für die Verwendung in Haftverbänden für die topische Anwendung an dem Körper geeignet sind, wobei das Verfahren umfasst:
Bereitstellen einer geschmolzenen Klebstoffzusammensetzung,
Kombinieren eines Inertgases mit der geschmolzenen Klebstoffzusammensetzung in einem ersten Mischer, der Mittel zum Mischen des Inertgases und des geschmolzenen Klebstoffs umfasst,
Mischen der geschmolzenen Klebstoffzusammensetzung, die das Inertgas umfasst, in dem Mischer unter Bedingungen, die wirksam sind, um eine erste geschäumte Klebstoffzusammensetzung zu bilden; und
Mischen der ersten geschäumten Klebstoffzusammensetzung in einem zweiten Mischer, der Mittel zum Mischen der ersten geschäumten Klebstoffzusammensetzung unter Bedingungen umfasst, die wirksam sind, um eine zweite geschäumte Klebstoffzusammensetzung zu bilden, die eine im Wesentlichen homogene Verteilung von geschlossenen Zellen aufweist, die das Inertgas darin enthalten umfassen,
wobei der Innendruck der ersten geschäumten Klebstoffzusammensetzung, die in den zweiten Mischer eintritt, größer als der Außendruck der zweiten geschäumten Klebstoffzusammensetzung ist, die aus dem zweiten Mischer austritt, um einen Druckunterschied zu liefern, wobei der Innendruck von 2,59 MPa (375 psi) bis 3,10 MPa (450 psi) beträgt und wobei der Außendruck von 1,21 MPa (175 psi) bis 2,24 MPa (325 psi) beträgt.

2. Verfahren gemäß Anspruch 1, wobei der Druckunterschied beträgt:
a) 0,79 MPa (115 psi) oder mehr.

3. Verfahren gemäß Anspruch 1, wobei der Innendruck von 2,76 MPa (400 psi) bis 2,93 MPa (425 psi) beträgt.

4. Verfahren gemäß Anspruch 1, wobei der Außendruck von 1,24 MPa (180 psi) bis 2,07 MPa (300 psi) beträgt.

5. Verfahren gemäß Anspruch 1, wobei der Druck des Inertgases, das in den ersten Mischer eintritt, höher als der Druck der geschmolzenen Klebstoffzusammensetzung, die in den ersten Mischer eintritt, ist.

6. Verfahren gemäß Anspruch 5, wobei der Druck des geschmolzenen Klebstoffs, der in den ersten Mischer eintritt, etwa gleich wie der Innendruck der ersten geschäumten Klebstoffzusammensetzung ist.

7. Verfahren gemäß Anspruch 1, wobei das Mittel zum Mischen der ersten Klebstoffzusammensetzung in dem zweiten Mischer eine rotierende Mischschaufel ist.

8. Verfahren gemäß Anspruch 7, wobei sich die rotierende Mischschaufel mit von etwa 20 bis etwa 30 min⁻¹ dreht.

9. Verfahren gemäß Anspruch 1, wobei die zweite geschäumte Klebstoffzusammensetzung eine berechnete Dichteabnahme von:
a) von 10 Prozent bis 50 Prozent; oder
b) von 20 Prozent bis 40 Prozent
aufweist.

10. Verfahren gemäß Anspruch 1, wobei die geschlossenen Zellen eine ovale, sphärische, kreisförmige oder langgestreckte Struktur aufweisen.

11. Verfahren gemäß Anspruch 1, wobei die geschlossenen Zellen eine Zellenabmessung ausgewählt aus der Gruppe bestehend aus mittlerem/mittlerer Durchmesser, Länge und Breite aufweisen, wobei die Zellabmessung vorzugsweise von 1 bis 10 Mikrometer beträgt.

12. Verfahren gemäß Anspruch 1, wobei die zweite geschäumte Klebstoffzusammensetzung eine Dichte von 0,14 bis 0,80 g/cm³ aufweist.

13. Verfahren gemäß Anspruch 1, wobei das Inertgas ausgewählt ist aus der Gruppe bestehend aus Stickstoff, Kohlendioxid, Edelgasen, Kohlenwasserstoffen und Luft.

14. Geschäumte Klebstoffzusammensetzung, umfassend eine im Wesentlichen homogene Verteilung von geschlossenen Zellen, die das Inertgas darin enthalten umfassen, hergestellt gemäß dem Verfahren gemäß Anspruch 1, wobei der Schaum eine Zellenabmessung ausgewählt aus der Gruppe bestehend aus mittlerem/mittlerer Durchmesser, Länge und Breite von 1 bis 10 Mikrometer aufweist.

## Revendications

1. Procédé de fabrication de compositions adhésives expansées aptes à une utilisation dans des pansements adhésifs destinés à une application topique sur le corps, le procédé comprenant :
la fourniture d'une composition adhésive fondue,
la combinaison d'un gaz inerte avec ladite composition adhésive fondue dans un premier mélangeur comprenant un moyen de mélange dudit gaz inerte et de l'adhésif fondu,
le mélange de ladite composition adhésive fondue comprenant ledit gaz inerte dans ledit mélangeur dans des conditions efficaces pour former une première composition adhésive expansée ; et
le mélange de ladite première composition adhésive expansée dans un deuxième mélangeur comprenant un moyen de mélange de ladite première composition adhésive expansée dans des conditions efficaces pour produire une deuxième composition adhésive expansée comportant une répartition sensiblement homogène d'alvéoles fermées comprenant ledit gaz inerte contenu dans lesdites alvéoles,
dans lequel une pression interne de ladite première composition adhésive expansée pénétrant dans ledit deuxième mélangeur est supérieure à une pression externe de ladite deuxième composition adhésive expansée sortant dudit deuxième mélangeur, en produisant ainsi une différence de pression, dans lequel ladite pression interne est de 2,59 MPa (375 livres par pouce carré (psi)) à 3,10 MPa (450 psi) et
dans lequel ladite pression externe est de 1,21 MPa (175 psi) à 2,24 MPa (325 psi).

2. Procédé selon la revendication 1, dans lequel ladite différence de pression est de :
a) 0,79 MPa (115 psi) ou plus.

3. Procédé selon la revendication 1, dans lequel ladite pression interne est de 2,76 MPa (400 psi) à 2,93 MPa (425 psi).

4. Procédé selon la revendication 1, dans lequel ladite pression externe est de 1,24 MPa (180 psi) à 2,07 MPa (300 psi).

5. Procédé selon la revendication 1, dans lequel la pression dudit gaz inerte pénétrant dans ledit premier mélangeur est supérieure à la pression de ladite composition adhésive fondue pénétrant dans ledit premier mélangeur.

6. Procédé selon la revendication 5, dans lequel ladite pression dudit adhésif fondu pénétrant dans ledit premier mélangeur est à peu près identique à ladite pression interne de ladite première composition adhésive expansée.

7. Procédé selon la revendication 1, dans lequel ledit moyen de mélange de ladite première composition adhésive dans ledit deuxième mélangeur est une palette de mélange rotative.

8. Procédé selon la revendication 7, dans lequel ladite palette de mélange rotative tourne à une vitesse d'environ 20 à environ 30 trs/min.

9. Procédé selon la revendication 1, dans lequel ladite deuxième composition adhésive expansée a une réduction de densité calculée de :
a) 10 % à 50 % ; ou
b) 20 % à 40 %.

10. Procédé selon la revendication 1, dans lequel lesdites alvéoles fermées ont une structure ovoïde, sphérique, circulaire ou allongée.

11. Procédé selon la revendication 1, dans lequel lesdites alvéoles fermées ont une dimension d'alvéole sélectionnée dans le groupe constitué d'un diamètre moyen, d'une longueur moyenne et d'une largeur moyenne, préférablement dans lequel ladite dimension d'alvéole est de 1 à 10 microns.

12. Procédé selon la revendication 1, dans lequel ladite deuxième composition adhésive expansée a une densité de 0,14 à 0,80 g/cm³.

13. Procédé selon la revendication 1, dans lequel ledit gaz inerte est sélectionné dans le groupe constitué de l'azote, du dioxyde de carbone, des gaz nobles, des hydrocarbures et de l'air.

14. Composition adhésive expansée comportant une répartition sensiblement homogène d'alvéoles fermées comprenant ledit gaz inerte contenu dans lesdites alvéoles, préparée par le procédé selon la revendication 1, dans laquelle la mousse a une dimension d'alvéole sélectionnée dans le groupe constitué d'un diamètre moyen, d'une longueur moyenne et d'une largeur moyenne de 1 à 10 microns.
